# EUROPÄISCHE PATENTANMELDUNG

(11) **EP 1 764 030 A1**
(43) Veröffentlichungstag der Anmeldung: **21.03.2007**
(21) Anmeldenummer: 05020420.5
(22) Anmeldetag: 20.09.2005
(51) Int. Cl.: A61B 5/00, G01N 33/487

(54) **Blutzuckermessgerät**

(71) Anmelder: F. HOFFMANN-LA ROCHE AG, 4070 Basel (CH); Roche Diagnostics GmbH, 68305 Mannheim (DE)
(72) Erfinder: Frikart, Marcel, 3012 Bern (CH); Jungen, Markus, 3065 Bolligen (CH); Haueter, Ulrich, 3506 Grosshöchstetten (CH)
(74) Vertreter: Blum, Rudolf Emil

(57) **Zusammenfassung**

Ein Blutzuckermessgerät (1) ist so ausgeführt, dass es an ein Mobiltelefon (2) ansteckbar ist, worauf die elektrische Speisung des Blutzuckermessgerätes vom Akku des Mobiltelefons übernommen wird. Dies erlaubt eine raum- und gewichtssparende Ausführung des Blutzuckermessgerätes.

## Beschreibung

Die Erfindung betrifft ein Blutzuckermessgerät und eine Einrichtung für Diabetespatienten. Ferner betrifft die Erfindung einen Satz von Blutzuckermessgeräten.

Blutzuckermessgeräte für Diabetiker zur regelmässigen Bestimmung des Blutzuckers sind bekannt. Bei der Messung wird der Blutzuckergehalt einer Blutprobe bestimmt, die sich der Diabetiker entnommen hat, wobei die Messung z.B. optisch oder elektrisch erfolgt. Das Blutzuckermessgerät zeigt den Messwert auf einer Anzeige an. Aus EP-B-0840465 ist es bekannt, dass ein Blutzuckermessgerät drahtlos Messewerte an ein tragbares elektrisches Gerät abgibt. Auf dem Markt erhältlich ist ein Mobiltelefon der Firma LG, Typ KP8400, das an seiner Rückseite integriert ein Blutzuckermessgerät aufweist.

Der Erfindung liegt die Aufgabe zu Grunde ein Blutzuckermessgerät zu verbessern.

Dies wird mit einem Blutzuckermessgerät gemäss Anspruch 1 erreicht.

Dadurch, dass das Blutzuckermessgerät zwar als separates Blutzuckermessgerät ausgeführt ist, es aber an ein anderes tragbares bzw. in der Hand haltbares Gerät, insbesondere ein Mobiltelefon, ansteckbar ist und seine elektrische Speisung aus diesem Gerät bezieht, kann ein sehr kleines, leichtes Blutzuckermessgerät geschaffen werden. Zwischen den Messungen kann das Blutzuckermessgerät vom Gerät bzw. Mobiltelefon getrennt werden und beeinträchtigt dann dessen Verwendung nicht.

Bevorzugterweise weist das Blutzuckermessgerät an seinem Gehäuse ein Adapterteil auf, das zur Steckverbindung an das jeweilige Gerät ausgebildet ist; dieser Adapterteil kann auch auswechselbar sein, um das Blutzukkermessgerät an verschiedene Geräte ansteckbar zu machen. Vorzugsweise erfolgt die Speisung des Blutzuckergerätes über die Ladebuchse des Gerätes und weiter ist es bevorzugt, wenn ein Datenaustausch zwischen dem Blutzuckermessgerät und dem Gerät erfolgt, vorzugsweise über eine Funkverbindung. Auf diese Weise kann die Anzeige des Gerätes vom Blutzuckermessgerät genutzt werden, sodass das Blutzuckermessgerät im Wesentlichen ohne Anzeigeeinrichtung auskommen kann, was weiter zu dessen Gewichts- bzw. Grössenreduktion führen kann. Bei einer Einrichtung für Diabetespatienten kann das Blutzuckermessgerät mit einer Insulinpumpe kommunizieren und/oder mit einer Fernsteuerung für das Blutzuckermessgerät.

Im Folgenden werden Ausführungsformen der Erfindung anhand der Figur näher erläutert. Dabei zeigt die
Figur 1 ein erstes Beispiel eines Blutzuckermessgerätes und eines Gerätes; und
Figur 2 ein zweites Beispiel eines Blutzukkermessgerätes und eines Gerätes.

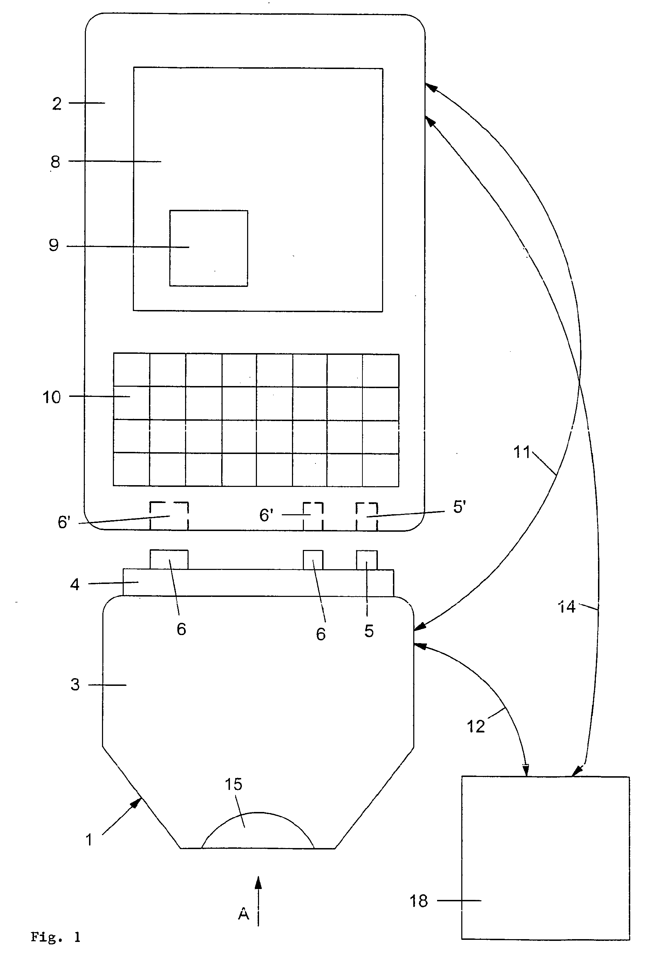

Figur 1 zeigt in schematischer Darstellung ein erstes Ausführungsbeispiel der Erfindung. Dabei ist ein Blutzuckermessgerät 1 vorgesehen, welches an ein tragbares elektronisches Gerät 2 angesteckt werden kann. Das tragbare elektronische Gerät 2 ist ein in der Hand tragbares Gerät (Handset) welches insbesondere ein Mobiltelefon oder ein Persönlicher Digitaler Assistent (PDA) ist. Entsprechend weist dieses Gerät 2 eine Anzeigeeinrichtung 8 und in der Regel eine Tastatur 10 auf, sowie eine in der Figur nicht dargestellte Stromversorgung, welche auf bekannte Weise einen aufladbaren elektrischen Akkumulator umfasst. Das Gerät 2 weist dabei - je nach Hersteller verschieden - eine Ladebuchse auf, an welcher der Stecker des Netzgerätes zur Aufladung des Akkumulators einsteckbar ist. In der Regel befindet sich diese Ladebuchse wie in der Figur 1 mit 5' angedeutet an der unteren Stirnseite des Gehäuses des elektronischen Gerätes 2.

Das Blutzuckermessgerät 1 ist nun derart ausgeführt, dass es an das elektronische Gerät 2 ansteckbar ist. Durch eine Steckverbindung kann somit das Blutzukkermessgerät 1 mit dem Gerät 2 verbunden werden, was in der Figur ersichtlich der Fall ist, wenn das Blutzuckermessgerät in Richtung des Pfeiles A bewegt wird. Das elektrische Steckkontaktteil 5 des Blutzuckermessgerätes 1 wird dabei in die Ladebuchse 5' eingeführt und die weiteren mechanischen Verbindungsmittel, insbesondere in der Form von Steckkontaktteilen 6 des Blutzuckermessgerätes 1 werden in Gehäuseausnehmungen 6' des Gerätes 2 eingeführt, sodass eine definierte mechanische Verbindung des Blutzuckermessgerätes 1 und des Gerätes 2 durch das Einstecken stattfindet und auch die elektrische Verbindung über den Steckkontakt 5 und die Ladebuchse 5' hergestellt wird. Vorausgesetzt ist dabei, dass an der Ladebuchse 5' die Stromversorgung des Gerätes 2 abgegriffen werden kann, sodass das Blutzuckermessgerät 1 in angesteckter Position durch die Stromversorgung des Gerätes 2 elektrisch gespiesen werden kann. Durch das Anstecken ist somit das Blutzuckermessgerät 1 mit elektrischer Speisung versorgt, sodass es eine Messung ausführen kann.

Die Art und Weise, wie das Blutzuckermessgerät den Blutzuckergehalt einer Blutprobe bestimmt, wird hier nicht weiter erläutert. Die Messprinzipien und die Konstruktion von Blutzuckermessgeräten sind dem Fachmann bekannt und müssen hier nicht erläutert werden. Das Blutzuckermessgerät 1 ist in diesem Aspekt ein konventionelles Blutzuckermessgerät, welches auf eine der bekannten Arten die Messung vornimmt.

Ersichtlich ist aus der vorgängigen Beschreibung, dass das Blutzuckermessgerät 1 keine eigene elektrische Stromquelle benötigt, da es ausschliesslich aus dem elektrischen Akkumulator des Gerätes 2 versorgbar ist. Das Blutzuckermessgerät kann dazu intern Spannungswandler aufweisen, um die elektronische Schaltung des Blutzuckermessgerätes an die Akkuspannung des Gerätes 2 anzupassen oder das Blutzuckermessgerät kann schaltungsmässig derart ausgelegt sein, dass es direkt mit dieser Akkuspannung betreibbar ist. Durch den Wegfall der eigenen Stromversorgung kann das Blutzuckermessgerät 1 deutlich kleiner und leichter ausgeführt sein, als dies bei herkömmlichen Blutzuckermessgeräten der Fall ist. Das Blutzuckermessgerät kann indes auch eine eigene Hilfsstromversorgung im Sinne eines kleinen Akkumulators oder eines Kondensators mit hoher Kapazität aufweisen, insbesondere um eine Messung oder eine Datenübergabe oder eine Datenspeicherung noch in geordnetem Zustand durchführen zu können, falls der Benutzer das Blutzuckermessgerät zu früh von dem elektronischen Gerät 2 trennt. Die Energiespeicherkapazität der Hilfsstromquelle ist somit vorzugsweise lediglich derart bemessen, dass ein geordnetes Abschalten des Blutzuckermessgerätes und/oder ein reduzierter Betrieb nach Trennung vom Gerät durchführbar ist, nicht aber der eigentliche Messbetrieb. Die Hilfstromquelle könnte aber auch grösser dimensioniert sein, so dass sie sogar den Messbetrieb zulässt. Sie kann ferner aus dem Gerät 2 aufladbar sein, was bevorzugt ist, oder sie kann aus einem separaten Ladegerät aufladbar sein.

Der Adapterteil 4 des Gehäuses 3 des Blutzukkermessgerätes ist an die jeweilige Ausgestaltung der elektrischen und mechanischen Kontaktmöglichkeiten des Gerätes 2 angepasst, sodass das Blutzuckermessgerät 1 mit verschiedenen Adaptern 4 erhältlich sein kann. Diese Adapter 4 können aber auch als austauschbare Adapter am Blutzuckermessgerät 1 vorgesehen sein, sodass durch Austausch des Adapters 4 eine Anpassung an ein anderes Gerät 2 möglich ist. So kann der Benutzer des Blutzuckermessgerätes dieses auch verwenden, wenn er ein Mobiltelefon 2 eines anderen Herstellers erwirbt. Es ist auch möglich, dass das Adapterteil 4 ein vom Gehäuse 3 des Blutzuckermessgerätes lösbares Adapterteil ist, welches dann mit dem Gehäuse 3 über eine kurze Kabelverbindung in elektrischem Kontakt steht. In diesem Fall wird nur der Adapterteil 4 an das elektronische Gerät 2 angesteckt und das Gehäuse 3 des Blutzuckermessgerätes ist dann mit dem Adapterteil über die erwähnte kurze Kabelverbindung verbunden.

Die mechanische Verbindung mit den mechanischen Steckkontaktteilen 6 erfolgt an entsprechenden Öffnungen 6' des Gehäuses des Gerätes 2, wobei diese Öffnungen oder Öffnung beim Gerät 2 eine Funktion als Datenschnittstelle haben kann, welche im gezeigten Beispiel von dem Blutzuckermessgerät nicht genutzt wird. Die Kontaktteile 6 sind in diesem Fall aus elektrisch nicht leitendem Material gebildet, sodass sie mit der Datenschnittstelle 6' keinen elektrischen Kontakt aufnehmen, sondern dort nur für den mechanischen Kontakt bzw. zur mechanischen Stabilisierung der Steckverbindung des Blutzuckermessgerätes 1 am Gerät 2 dienen.

Das Blutzuckermessgerät 1 kann derart ausgestattet sein, dass es automatisch den Betrieb aufnimmt, sobald es über das Gerät 2 mit der Speisespannung versorgt wird. Der Benutzer kann damit nach dem Anstecken des Blutzuckermessgerätes 1 die Blutprobe, zum Beispiel auf einem Messstreifen oder dergleichen, in einen Messbereich 15 des Blutzuckermessgerätes 1 einbringen und die Messung durchführen lassen. Das Blutzuckermessgerät kann dann die Messwerte auf einer eigenen Anzeigevorrichtung darstellen, welche in der Figur nicht gezeigt ist. Bevorzugterweise erfolgt nämlich ein Datenaustausch mit dem Gerät 2 derart, dass mindestens die Anzeigevorrichtung 8 zur Darstellung der Messwerte des Blutzuckermessgerätes 1 verwendet wird. Vorzugsweise erfolgt die Datenverbindung des Blutzuckermessgerätes 1 mit dem elektronischen Gerät 2 über eine drahtlose Verbindung 11, welche insbesondere eine Funkverbindung nach dem Bluetooth-Standard ist. Entsprechend ist im Blutzuckermessgerät 1 ein Bluetooth-Modul vorgesehen, welches die Kommunikation des Blutzukkermessgerätes mit einem anderen Bluetooth-Gerät ausführen kann. Das in der Hand haltbare Gerät 2 muss in diesem Fall ebenfalls eine Bluetooth-Funktionalität aufweisen, was bei modernen Mobiltelefonen (Smartphones) oder Persönlichen Digitalen Assistenten (PDA) der Fall ist. Auf diese Weise kann das Blutzuckermessgerät die Messdaten auf das Gerät 2 übertragen, wobei dieses natürlich entsprechend eingerichtet sein muss und auf ihm ein Programm ausgeführt werden muss, welches die Darstellung der Messwerte ermöglicht. Diese können zum Beispiel in einem separaten Fenster 9 der Anzeige 8 des Gerätes 2 dargestellt werden.

Der Vorgang bei einer Blutzuckerbestimmung des Diabetespatienten läuft damit derart ab, dass der Patient sein Gerät bzw. Handset 2 zur Hand nimmt, welches eingeschaltet ist. Danach steckt er das Blutzuckermessgerät an dem Gerät 2 an, wie dies erläutert worden ist. Damit erfolgt bevorzugterweise ein automatischer Start-up des Blutzuckermessgerätes. In der Zwischenzeit kann der Patient die für die Messung benötigte Blutprobe entnehmen. Nach der Initialisierung des Blutzuckermessgerätes erfolgt bevorzugterweise der automatische Verbindungsaufbau des Bluetooth-Modules des Messgerätes zu dem Handset 2. Auf diesem erfolgt danach der automatische Start der entsprechenden Applikation, die die Kommunikation mit dem Blutzuckermessgerät und die Darstellung von dessen Messwerten erlaubt. Es kann eine Anzeige vorgesehen sein, die dem Benutzer zeigt, dass das System aus Blutzuckermessgerät und Handset 2 für die Messung bereit ist. Diese Anzeige kann z.B. auf der Anzeige 8 des Gerätes 2 erfolgen oder kann eine akustische Anzeige dieses Gerätes sein oder kann z.B. durch eine Statusanzeige, welche eine farbige LED sein kann, auf dem Blutzuckermessgerät erfolgen. Danach erfolgt die Messung des Blutzuckerwertes und nach dieser die Anzeige, dass die Messung abgeschlossen worden ist, was ebenfalls wieder an dem Gerät 2 und/oder an dem Blutzuckermessgerät 1 erfolgen kann. Der ermittelte Messwert wird danach automatisch via Bluetooth auf das Gerät 2 übertragen und dort auf der Anzeigeeinrichtung 8, z.B. in einem separaten Fenster 9, dargestellt. Eventuell kann eine zweite Messung durchgeführt werden und nachfolgend wird das Blutzuckermessgerät vom Gerät 2 gelöst und es ergibt sich ein automatischer Verbindungsabbau und ein geordnetes Abschalten des Blutzuckermessgerätes durch die darin bevorzugt angeordnete Hilfsstromversorgung, welche, wie erwähnt, für diese Vorgänge genügend Energie bereitstellt, aber nicht für die eigentliche Messung als Stromversorgung vorgesehen ist, sodass eine Messung ohne das Anstecken am Gerät 2 nicht ausführbar ist. Der Patient kann nun mit den gemessenen Werten seine Insulinbehandlung durchführen.

Das System aus Blutzuckermessgerät und Gerät 2 kann aber auch Teil einer Einrichtung für Diabetespatienten bzw. eines Diabetesmanagementsystems sein, indem die Messwerte automatisch an eine Insulinpumpe 18 abgegeben werden. Dies kann einerseits über das Bluetooth-Modul des Blutzuckermessgerätes erfolgen, solange dieses an dem Gerät 2 angekoppelt und von diesem gespiesen wird, was durch den Pfeil 12 in der Figur 1 angedeutet ist. Die Insulinpumpe 18 kann aber auch über das Bluetooth-Modul des Gerätes 2 angesprochen werden, was durch den Verbindungspfeil 14 dargestellt ist. Dies kann dann auch erfolgen, wenn das Blutzuckermessgerät bereits wieder vom Gerät 2 abgekoppelt ist.

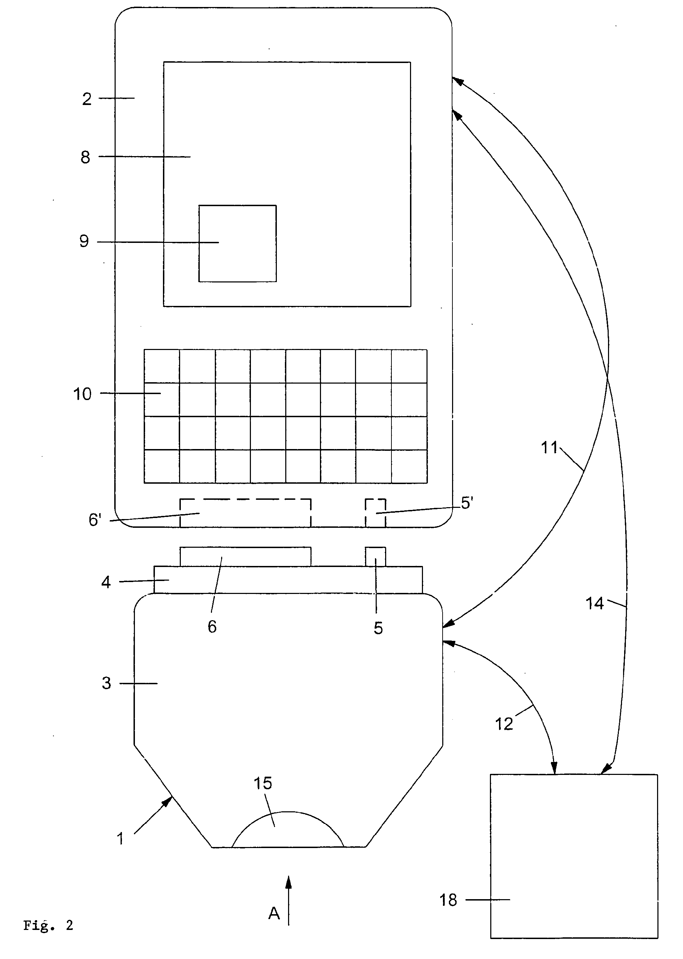

Figur 2 zeigt eine weitere Ausführungsform, bei welcher für gleiche Elemente gleiche Bezugszeichen verwendet worden sind. Die in Figur 2 dargestellte Ausführungsform unterscheidet sich lediglich in der Ausgestaltung des Adapters 4, welcher einen anders geformten Stecker 5 zur elektrischen Kontaktnahme und einen anders geformten elektrisch neutralen mechanischen Steckverbinder 6 aufweist.

Anstelle der Stromversorgung über die Ladebuchse des Gerätes 2 könnte bei beiden Beispielen auch eine Stromversorgung über eine drahtgebundene Geräteschnittstelle des Gerätes 2 erfolgen, wenn dieses mit einem solchen Interface ausgestaltet ist und der Adapter 4 entsprechend zur Abnahme der Betriebsspannung für das Blutzuckermessgerät an diesem Interface ausgestaltet ist. Bei modernen Mobiltelefonen können entsprechende Interfaces für Kameras oder andere Zubehöre vorgesehen sein, welche neben der Betriebsspannungsausgangsbereitstellung auch zum Beispiel einen USB-Port bereitstellen. Auch können angeschlossene Geräte und somit insbesondere das Blutzuckermessgerät über dieses Interface identifiziert werden und das Gerät 2 kann die auf ihm laufenden Applikationen entsprechend auswählen. Für den Fall eines Gerätes 2 mit einer derartigen Schnittstelle ist es auch möglich den Datenaustausch des Blutzuckermessgerätes anstelle der drahtlosen Kommunikation und insbesondere der Bluetooth-Kommunikation über dieses Interface durchzuführen.

Als elektronisches Gerät 2 kommt, wie bereits erwähnt, insbesondere ein Mobiltelefon und insbesondere ein Smartphone oder ein Persönlicher Digitaler Assistent (PDA) in Frage, welches Gerät 2 insbesondere mit einem programmierbaren Betriebssystem, zum Beispiel Palm OS Windows OS, Symbian OS oder Java-Umgebung ausgestattet sein sollte. Ferner benötigt das Handset 2 für die bevorzugte Datenübergabe eine bluetooth Schnittstelle und vorzugsweise das bluetooth serial port profile (SPP) oder FTP, OBEX, PAN, Object exchange oder ein anderes Profil.

Anstelle oder zusätzlich zur Insulinpumpe 18 könnte auch im Sinne der erwähnten Einrichtung für Diabetespatienten eine weitere bzw. andere Nebenstelle vorgesehen sein, die in den Zeichnungen nicht dargestellt ist, und die drahtlos, insbesondere über die Bluetooth-Verbindung oder zum Beispiel über eine Infrarotverbindung, mit dem Blutzuckermessgerät in Verbindung steht und für dieses eine Bedienungseinrichtung und/oder auch eine Anzeigeeinrichtung bildet, so dass eine Bedienung über einen an der Nebenstelle vorgesehenen Bedienungsknopf oder Bedienungsknöpfe erfolgen kann und oder dass Anzeigen, insbesondere Statusanzeigen des Blutzuckermessgerätes über die Nebenstelle erfolgen können. Diese kann damit insbesondere eine Fernsteuerung für das Blutzuckermessgerät bilden, so dass das am Gerät 2 angeschlossene Blutzuckermessgerät fernbedient werden kann.

Durch die Erfindung kann ein sehr kleines und sehr leichtes Blutzuckermessgerät bereitgestellt werden, welches für die Messung die gleiche Funktionalität aufweist wie die bekannten Blutzuckermessgeräte. Beim Blutzuckermessgerät selber muss aber keine Batterie gewechselt werden oder aufgeladen werden. Es kann ein vollautomatisches Messverfahren erfolgen, das kein Einschalten, kein Ausschalten und keine Auslösung für die Datenübertragung benötigt, da dies beim Anstecken automatisch erfolgt. Das Gerät 2 kann eine Messwertanzeige auf einem grossen Farbdisplay ermöglichen oder es kann ein komplettes Userinterface auf die Anzeige 2 ausgelagert werden. Zusätzliche Anwendungen sind auf dem Gerät 2 möglich, zum Beispiel komfortable Erinnerungsfunktionen an eine notwendige Blutzuckermessung. Es ist auch eine direkte Integration in ein bestehendes Diabetesmanagementsystem möglich.

## Patentansprüche

1. Blutzuckermessgerät (1), **dadurch gekennzeichnet, dass** dieses an ein tragbares elektronisches Gerät (2) ansteckbar und von diesem wieder lösbar ist, wobei das Gerät (2) eine aufladbare elektrische Stromquelle und eine Anzeigevorrichtung umfasst, und wobei durch das Anstecken die elektrische Verbindung des Blutzuckermessgerätes zur Stromquelle des Gerätes hergestellt wird, derart, dass das angesteckte Blutzuckermessgerät zur Ausführung des Messbetriebes von der Stromquelle speisbar ist.

2. Blutzuckermessgerät nach Anspruch 1 **dadurch gekennzeichnet, dass** dieses ein Gehäuse (3) mit einem gerätespezifischen Adapterteil (4) aufweist, welcher steckbare elektrische Kontaktmittel (5) und zusätzlich mindestens ein steckbares mechanisches Verbindungsmittel (6) aufweist.

3. Blutzuckermessgerät nach Ansprüchen 1 oder 2 **dadurch gekennzeichnet, dass** das Gerät ein Mobiltelefon (2) oder ein Persönlicher Digitaler Assistent (PDA) ist.

4. Blutzuckermessgerät nach einem der Ansprüche 1 bis 3 **dadurch gekennzeichnet, dass** die elektrische Speisung des Blutzuckermessgerätes über die Ladebuchse des Gerätes erfolgt.

5. Blutzuckermessgerät nach einem der Ansprüche 1 bis 3 **dadurch gekennzeichnet, dass** die elektrische Speisung des Blutzuckermessgerätes über eine drahtgebundene Geräteschnittstelle des Gerätes erfolgt.

6. Blutzuckermessgerät nach einem der vorangehenden Ansprüche **dadurch gekennzeichnet, dass** dieses zum Datenaustausch mit dem Gerät über eine drahtlose Schnittstelle, bevorzugterweise über eine Funkschnittstelle, verfügt, insbesondere über ein Bluetooth-Modul.

7. Blutzuckermessgerät nach einem der vorangehenden Ansprüche **dadurch gekennzeichnet, dass** dieses zum Datenaustausch mit dem Gerät über eine parallele Datenschnittstelle und/oder eine serielle Datenschnittstelle verfügt.

8. Blutzuckermessgerät nach einem der vorangehenden Ansprüche **dadurch gekennzeichnet, dass** es keine numerische und/oder alphanumerische Anzeige aufweist.

9. Blutzuckermessgerät nach einem der vorangehenden Ansprüche **dadurch gekennzeichnet, dass** es keine eigene elektrische Stromquelle für den Messbetrieb aufweist.

10. Blutzuckermessgerät nach einem der vorangehenden Ansprüche, **dadurch gekennzeichnet, dass** es eine eigene Hilfsstromversorgung, insbesondere einen Akkumulator oder einen Kondensator mit hoher Kapazität aufweist, deren Energiespeicherkapazität derart bemessen ist, dass ein geordnetes Abschalten des Blutzuckermessgerätes oder ein reduzierter Betrieb nach Trennung vom Gerät durchführbar ist.

11. Einrichtung für Diabetes-Patienten mit einem Blutzuckermessgerät nach einem der Ansprüche 1 bis 10 sowie mindestens einem mit dem Blutzuckermessgerät und/oder dem Gerät (2) kommunizierendem, insbesondere drahtlos kommunizierendem, Apparat (18), der insbesondere eine Insulinpumpe ist oder der insbesondere eine Fernsteuerung für das Blutzuckermessgerät ist.

12. Satz von Blutzuckermessgeräten nach einem der Ansprüche 1 bis 10 **dadurch gekennzeichnet, dass** diese mit verschiedenen gerätespezifischen Adapterteilen (4) ausgerüstet sind.
